(19) Europäisches Patentamt
European Patent Office
Office européen des brevets

(11) EP 3 957 631 B1

(12) **EUROPEAN PATENT SPECIFICATION**

(45) Date of publication and mention of the grant of the patent:
**10.09.2025 Bulletin 2025/37**

(21) Application number: **20790646.2**

(22) Date of filing: **20.04.2020**

(51) International Patent Classification (IPC):
***A61K 31/4709*** *(2006.01)* ***A61K 31/475*** *(2006.01)*
***A61K 31/4745*** *(2006.01)* ***A61K 45/06*** *(2006.01)*
***A61P 35/00*** *(2006.01)* ***C07D 401/12*** *(2006.01)*

(52) Cooperative Patent Classification (CPC):
(C-Sets available)
**A61P 35/00; A61K 31/4709; A61K 31/4745; A61K 31/475; A61K 45/06; C07D 401/12** (Cont.)

(86) International application number:
**PCT/CN2020/085552**

(87) International publication number:
**WO 2020/211860 (22.10.2020 Gazette 2020/43)**

(54) **QUINOLINE COMPOUND OR PHARMACEUTICALLY ACCEPTABLE SALT THEREOF FOR TREATING EWING'S SARCOMA**

CHINOLINVERBINDUNG ODER PHARMAZEUTISCH ANNEHMBARES SALZ DAVON ZUR BEHANDLUNG DES EWING-SARKOMS

COMPOSÉ DE QUINOLÉINE OU SEL PHARMACEUTIQUEMENT ACCEPTABLE DE CELUI-CI POUR LE TRAITEMENT DU SARCOME D'EWING

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2019 CN 201910315417**

(43) Date of publication of application:
**23.02.2022 Bulletin 2022/08**

(73) Proprietor: **Chia Tai Tianqing Pharmaceutical Group Co., Ltd.**
**Lianyungang City, Jiangsu 222062 (CN)**

(72) Inventors:
- **ZHAN, Xiaole**
**Lianyungang City, Jiangsu 222062 (CN)**
- **TU, Lifan**
**Lianyungang City, Jiangsu 222062 (CN)**
- **YANG, Chaoqiang**
**Lianyungang City, Jiangsu 222062 (CN)**
- **ZHANG, Xiquan**
**Lianyungang City, Jiangsu 222062 (CN)**
- **WANG, Xunqiang**
**Lianyungang City, Jiangsu 222062 (CN)**
- **XU, Jie**
**Beijing 100044 (CN)**
- **XU,Ping**
**Nanjing, Jiangsu 211100 (CN)**
- **WANG,Chengqian**
**Nanjing, Jiangsu 211100 (CN)**

(74) Representative: **Geling, Andrea**
**ZSP Patentanwälte PartG mbB**
**Hansastraße 32**
**80686 München (DE)**

(56) References cited:
**CN-A- 105 311 029** **CN-A- 105 311 030**
**CN-A- 107 771 078** **CN-A- 107 970 241**

- **ANONYMOUS: "Anlotinib and Irinotecan for Ewing Sarcoma - Full Text View - ClinicalTrials.gov", 15 February 2019 (2019-02-15), XP093001900, Retrieved from the Internet <URL:https://clinicaltrials.gov/ct2/show/NCT03416517> [retrieved on 20221124]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PRETZ J ET AL: "Localized Adult Ewing Sarcoma (ES): Improved Outcomes With Ifosfamide and Etoposide (IE) Added to Cyclophosphamide, Doxorubicin, and Vincristine (CAV) Chemotherapy (CT)", INTERNATIONAL JOURNAL OF RADIATION: ONCOLOGY BIOLOGY PHYSICS, vol. 90, no. 1, 1 January 2014 (2014-01-01), XP029059349, ISSN: 0360-3016, DOI: 10.1016/J.IJROBP.2014.05.2182**
- **DENG, XUESONG: "Diagnosis and Treatment Progress of Ewing Sarcoma", CHINESE GENERAL PRACTICE, vol. 17, no. 12, 30 April 2014 (2014-04-30), CN , pages 1334 - 1337, XP009532167, ISSN: 1007-9572, DOI: 10.3969/ j.issn.1007-9572.2014.12.002**
- **WANG GANGYANG ET AL.: "Anlotinib, A Novel Small Molecular Tyrosine Kinase Inhibitor, Suppresses Growth and Metastasis via Dual Blockade of VEGFR2 and MET in Osteosarcoma", INT. J. CANCER, vol. 145, no. 4, 4 February 2019 (2019-02-04), XP055743777, ISSN: 1097-0215, DOI: 20200604165842X**
- **SHEN GUOSHUANG ET AL.: "Anlotinib: A Novel Multi-Targeting Tyrosine Kinase Inhibitor in Clinical Development", JOURNAL OF HEMATOLOGY & ONCOLOGY, vol. 11, no. 1, 19 September 2018 (2018-09-19), XP055700540, ISSN: 1756-8722, DOI: 20200604170538X**
- **TANG L. ET AL.: "Anlotinib Inhibits Synovial Sarcoma by Targeting GINS1: A Novel Downstream Target Oncogene in Progression of Synovial Sarcoma", CLINICAL AND TRANSLATIONAL ONCOLOGY, vol. 21, no. 12, 8 April 2019 (2019-04-08), XP036935883, ISSN: 1699-048X, DOI: 20200604171002X**
- **HUIFENG ZHOU; CHAO LU: "Diagnosis and treatment progress of Ewing Sarcoma", INTERNATIONAL JOURNAL OF PEDIATRICS, vol. 44, no. 3, 31 March 2017 (2017-03-31), pages 196 - 199, XP009532190, ISSN: 1673-4408, DOI: 10.3760/cma.j.issn.1673-4408.2017.03.013**
- **SUN YUAN-JUE,ZHANG JIAN-JUN,HE AI-NA,ZHENG SHUI-ER,SHEN ZAN,YAO YANG: "Clinical observation of cyclophosphamide combined with hydroxycamptothecin as second-line treatment on advanced Ewing's sarcoma", CHINESE CLINICAL ONCOLOGY, vol. 16, no. 11, 15 November 2011 (2011-11-15), pages 1016 - 1019, XP009532130, ISSN: 1009-0460**


(52) Cooperative Patent Classification (CPC): (Cont.)

C-Sets
**A61K 31/4709, A61K 2300/00;**
**A61K 31/4745, A61K 2300/00;**
**A61K 31/475, A61K 2300/00**

## Description

### TECHNICAL FIELD

The present invention relates to the field of medicines, and particularly relates to a quinoline compound or a pharmaceutically acceptable salt thereof for use in treating Ewing's sarcoma and a quinoline compound or a pharmaceutically acceptable salt thereof for combined treatment of Ewing's sarcoma.

### BACKGROUND

Currently, Ewing's sarcoma is recognized as an independent bone tumor, and it is defined as a small round cell tumor of bone or soft tissue originating in neuroectoderm. Ewing's sarcoma accounts for 6%-8% of all primary bone tumors and 10%-14.2% of malignant bone tumors, and is second only to osteosarcoma and chondrosarcoma. It is the most common malignant primary bone tumor in children and adolescents, and is slightly more likely to be found in males than females.

The most common early symptoms of Ewing's sarcoma are pain and swelling, with more than 60% of patients presenting intermittent pain in the early stages and the pain sites spreading with tumor spread. Local lumps appear along with the aggravation of pain, and the patient experiences obvious pain when the lumps are pressed. Besides, symptoms of nerve function injury in nerve root, spinal cord, etc., appear. Some patients have fever at 38-40 °C, and serum high density lipoprotein, cholesterol and red blood cell sedimentation rate increase significantly, accompanied by increased white blood cell count and anemia. Lesions can produce large soft tissue lumps. Because Ewing's sarcoma features high malignancy degree and rapid metastasis, the effect is not ideal when it is treated with only surgery, radiotherapy or single-drug chemotherapy, and the 5-year survival rate is no more than 10%.

At present, the more effective treatment method for Ewing's sarcoma is a combination therapy, i.e. a combination therapy of radiotherapy with chemotherapy and surgery (or without surgery), wherein for chemotherapy, multi-drug combination chemotherapy is the most common. (Deng, Chinese General Practice, vol. 17, no. 12, 2014). Pretz et al. (INTERNATIONAL JOURNAL OF RADIATION: ONCOLOGY BIOLOGY PHYSICS, Vol 90, Issue 1, 2014) describes a combination chemotherapy comprising ifosfamide and etoposide added to cyclophosphamide, doxorubicin and vincristine for treating localized Ewing's sarcoma.

Although research shows that the 5-year survival rate of Ewing's sarcoma can be remarkably improved by multi-drug combination chemotherapy and the like, the chemotherapy for Ewing's sarcoma generally lasts for 2 years because it mostly metastasizes within 2 years, and long-term chemotherapy results in toxic and side effects, such as complications of hematopoietic system, respiratory system, cardiovascular system, nervous system and urinary system. Therefore, there is an urgent need to develop new therapeutic regimens for Ewing's sarcoma.

### SUMMARY

The invention is set out in the appended set of claims. Specifically, in a first aspect, the present invention provides a compound of formula I or a pharmaceutically acceptable salt thereof for use in treating Ewing's sarcoma.

The chemical name of the compound of formula I is 1-[[[4-(4-fluoro-2-methyl-1*H*-indol-5-yl)oxy-6-methoxyquinolin -7-yl]oxy]methyl]cyclopropylamine, which has the following structural formula:

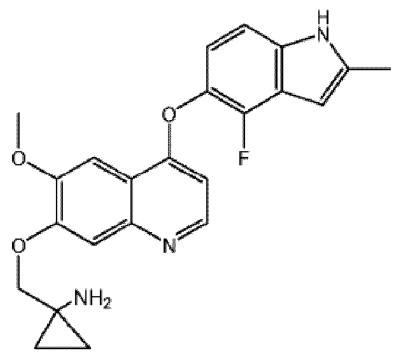

formula I.

In some embodiments, the Ewing's sarcoma is endoskeletal Ewing's sarcoma, extraskeletal Ewing's sarcoma or periosteal Ewing's sarcoma.

In some embodiments, the Ewing's sarcoma is osteolytic Ewing's sarcoma, sclerosing Ewing's sarcoma or

hybrid Ewing's sarcoma.

In some embodiments, the Ewing's sarcoma is advanced Ewing's sarcoma. In some embodiments, advanced Ewing's sarcoma refers to primary or locally recurrent Ewing's sarcoma that cannot be eradicated by surgery or other local treatment, or that the patient refuses to treat with surgery or other local treatment. In some embodiments, advanced Ewing's sarcoma refers to one that, in the case of distal metastasis, cannot be eradicated by surgery or other local treatment, or that the patient refuses to treat with surgery or other local treatment.

In some embodiments, the Ewing's sarcoma is one that is after failure of a prior treatment. Preferably, the Ewing's sarcoma is one that is after failure of treatment with radiotherapy and/or a chemotherapeutic drug. More preferably, the Ewing's sarcoma is one that is after failure of treatment with the chemotherapeutic agent selected from one or more of cyclophosphamide, ifosfamide, doxorubicin, pirarubicin, dactinomycin, bleomycin, vincristine, carmustine, etoposide, actinomycin D, methotrexate and cisplatin. Most preferably, the chemotherapeutic agent that the subject with Ewing's sarcoma has received is selected from one or more of cyclophosphamide, ifosfamide, doxorubicin, pirarubicin, vincristine and etoposide.

The compound of formula I described herein can be administered in its free base form, or in the form of a salt, a hydrate, or a prodrug that may convert *in vivo* into the free base form of the compound of formula I. For example, within the scope of the present invention, the pharmaceutically acceptable salt of the compound of formula I can be generated from various organic and inorganic acids according to methods commonly known in the art.

In some embodiments, the pharmaceutically acceptable salt of the compound of formula I includes, but is not limited to, salts formed by the compound of formula I and any of the following acids: hydrochloric acid, hydrobromic acid, sulfuric acid, nitric acid, phosphoric acid, acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid and stearic acid. In the present invention, the pharmaceutically acceptable salt of the compound of formula I is preferably in the form of hydrochloride or maleate, more preferably dihydrochloride.

In some embodiments, the compound of formula I or the pharmaceutically acceptable salt thereof is administered in the form of a hydrochloride. In some embodiments, the compound of formula I or the pharmaceutically acceptable salt thereof is administered in the form of a monohydrochloride. In some embodiments, the compound of formula I or the pharmaceutically acceptable salt thereof is administered in the form of a dihydrochloride. In some embodiments, the compound of formula I or the pharmaceutically acceptable salt thereof is administered in the form of a crystalline hydrochloride. In some embodiments, the compound of formula I or the pharmaceutically acceptable salt thereof is administered in the form of a crystalline dihydrochloride. In some embodiments, the compound of formula I or the pharmaceutically acceptable salt thereof is administered in the form of a maleate.

Described herein, but not encompassed by the present invention, is a method for use in treating Ewing's sarcoma, which comprises administering to a patient in need thereof a therapeutically effective amount of the compound of formula I or the pharmaceutically acceptable salt thereof.

The compound of formula I or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations. In a specific embodiment, the drug is administered orally.

The dosage regimen of the compound of formula I can be determined comprehensively depending on the activity and toxicity of the drug, tolerance of a patient, etc.

In some embodiments, the compound of formula I or the pharmaceutically acceptable salt thereof can be provided in the form of a formulation suitable for any one of oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations, preferably in the form of a formulation suitable for oral administration. In some embodiments, the compound of formula I or the pharmaceutically acceptable salt thereof can be provided preferably in the form of tablets, capsules, powders, granules, dripping pills, pastes or pulvis, more preferably tablets or capsules.

In some embodiments, the administered amount of the compound of formula I or the pharmaceutically acceptable salt thereof can be determined according to the severity of a disease, the response of the disease, any treatment-related toxicity, and the age and health of a patient. In some embodiments, the compound of formula I or the pharmaceutically acceptable salt thereof is administered at a daily dose of 3 mg to 30 mg, preferably 5 mg to 20 mg, more preferably 8 mg to 16 mg, further preferably 8 mg to 14 mg, and most preferably 8 mg, 10 mg, or 12 mg.

Preferably, the compound of formula I or the pharmaceutically acceptable salt thereof is administered in an intermittent regimen. The intermittent regimen includes treatment periods and interruption periods. In the treatment

period, the compound of formula I or the pharmaceutically acceptable salt thereof can be administered once or multiple times daily. For example, the compound of formula I or the pharmaceutically acceptable salt thereof is administered daily in the treatment period, and then the treatment is interrupted during the interruption period, followed by the treatment period and then the interruption period, over and over again. The ratio of the treatment period to the interruption period in days is 2:0.5-2:5, preferably 2:0.5-2:3, more preferably 2:0.5-2:2, and even more preferably 2:0.5-2:1.

As a still further preferred intermittent regimen, the intermittent regimen may be selected from one of the following cycles: consecutively 2-week treatment and then 2-week interruption, consecutively 2-week treatment and then 1-week interruption, and consecutively 5-day treatment and then 2-day interruption, wherein the cycle can be repeated multiple times.

In yet another aspect, the present invention provides a pharmaceutical composition for use in treating Ewing's sarcoma, which comprises the compound of formula I or the pharmaceutically acceptable salt thereof, and at least one pharmaceutically acceptable carrier.

The pharmaceutical composition includes, but is not limited to, a preparation suitable for oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations, preferably a preparation suitable for oral administration, including tablets, capsules, powders, granules, dripping pills, pastes and pulvis, and more preferably tablets or capsules. The tablet may be a common tablet, dispersible tablet, effervescent tablet, sustained-release tablet, controlled-release tablet or enteric coated tablet. The capsule may be a common capsule, sustained-release capsule, controlled-release capsule or enteric coated capsule. The oral preparation may be prepared by a conventional method using a pharmaceutically acceptable carrier well known in the art. The pharmaceutically acceptable carrier includes fillers, absorbents, wetting agents, binders, disintegrants, lubricants, and the like. The fillers include starch, lactose, mannitol, microcrystalline cellulose, and the like. The absorbents include calcium sulfate, calcium hydrogen phosphate, calcium carbonate, and the like. The wetting agents include water, ethanol, and the like. The binders include hydroxypropyl methylcellulose, polyvidone, microcrystalline cellulose, and the like. The disintegrants include croscarmellose sodium, crospovidone, surfactants, low-substituted hydroxypropyl cellulose, and the like. The lubricants include magnesium stearate, talcum powder, polyethylene glycol, sodium dodecyl sulfate, colloidal silicon dioxide, talcum powder, and the like. The pharmaceutically acceptable excipient further includes coloring agents, sweeteners and the like.

In one embodiment, the pharmaceutical composition is a solid preparation suitable for oral administration. The composition, for example, may be in the form of a tablet or capsule. In one specific embodiment, the pharmaceutical composition is a capsule. In one specific embodiment of the present invention, the pharmaceutically acceptable carrier of the oral solid preparation comprises mannitol, microcrystalline cellulose, hydroxypropylcellulose, and magnesium stearate.

Described herein, but not encompassed by the present invention, is the use of a compound of formula I or a pharmaceutically acceptable salt thereof in combination with a second therapeutic agent in preparing a combination for treating Ewing's sarcoma.

In some embodiments, the Ewing's sarcoma includes, but is not limited to, endoskeletal Ewing's sarcoma, extraskeletal Ewing's sarcoma or periosteal Ewing's sarcoma.

In some embodiments, the Ewing's sarcoma includes, but is not limited to, osteolytic Ewing's sarcoma, sclerosing Ewing's sarcoma or hybrid Ewing's sarcoma.

In some embodiments, the Ewing's sarcoma is advanced Ewing's sarcoma. In some embodiments, advanced Ewing's sarcoma refers to primary or locally recurrent Ewing's sarcoma that cannot be eradicated by surgery or other local treatment, or that the patient refuses to treat with surgery or other local treatment. In some embodiments, advanced Ewing's sarcoma refers to one that, in the case of distal metastasis, cannot be eradicated by surgery or other local treatment, or that the patient refuses to treat with surgery or other local treatment.

In some embodiments, the Ewing's sarcoma includes, but is not limited to, one that is after failure of a prior treatment. Preferably, the Ewing's sarcoma is one that is after failure of treatment with radiotherapy and/or a chemotherapeutic drug. More preferably, the chemotherapeutic agent that a subject with Ewing's sarcoma has received is selected from one or more of cyclophosphamide, ifosfamide, doxorubicin, pirarubicin, dactinomycin, bleomycin, vincristine, carmustine, etoposide, actinomycin D, methotrexate and cisplatin. Most preferably, the chemotherapeutic agent that the subject with Ewing's sarcoma has received is selected from one or more of cyclophosphamide, ifosfamide, doxorubicin, pirarubicin, vincristine and etoposide.

In some embodiments, the second therapeutic agent is a chemotherapeutic drug, including, but not limited to, one or more of an alkylating agent, a podophyllum, a camptothecin analog, a taxane, an antimetabolite and an anti-tumor antibiotic. Examples that may be listed include, but are not limited to, one or more of a platinum-based drug (e.g., oxaliplatin, cisplatin, carboplatin, nedaplatin, and dicycloplatin), a fluoropyrimidine derivative (e.g., gemcitabine, capecitabine, fluorouracil, difuradin, doxifluridine, tegafur, carmofur, and trifluridine), a taxane (e.g., paclitaxel, albumin-bound paclitaxel, and docetaxel), a camptothecin analog (e.g., camptothecin, hydroxycamptothecin, irinotecan, and topotecan), a vinca alkaloid (vinorelbine, vinblastine, vincristine, vindesine, and vinflunine), pemetrexed, carmustine, etoposide,

teniposide, mitomycin, ifosfamide, cyclophosphamide, azacitidine, doxorubicin, pirarubicin, amrubicin, methotrexate, bendamustine, epirubicin, actinomycin D, dactinomycin, bleomycin, temozolomide, LCL-161, KML-001, sapacitabine, plinabulin, treosulfan, tipiracil hydrochloride, $^{153}$Sm-EDTMP, tegafur-gimeracil-oteracil potassium, and encequidar. In some embodiments, the chemotherapeutic drug is selected from one or more of vincristine, cyclophosphamide, ifosfamide, doxorubicin, dactinomycin, bleomycin, carmustine, etoposide, actinomycin D, methotrexate, and cisplatin.

As required, the second therapeutic agent may be further used in combination with an ancillary drug for chemotherapy. The ancillary drug for chemotherapy includes but is not limited to calcium folinate (CF), leucovorin, mesna, bisphosphonate, amifostine, and hematopoietic cell colony stimulating factors (CSFs). In some embodiments, the ancillary drug for chemotherapy is CF, mesna, and leucovorin.

In some embodiments, the second therapeutic agent is a combination of camptothecin analogs and vinca alkaloids. In some embodiments, the second therapeutic agent is a combination of one of the camptothecin analogs and one of the vinca alkaloids. In some embodiments, the camptothecin analogs are selected from one or more of camptothecin, hydroxycamptothecin, irinotecan, and topotecan; the vinca alkaloids are selected from one or more of vinorelbine, vinblastine, vincristine, vindesine, and vinflunine.

In some embodiments, the second therapeutic agent is a combination of vinca alkaloids and one selected from the group consisting of camptothecin, hydroxycamptothecin, irinotecan, and topotecan. In some embodiments, the second therapeutic agent is a combination of camptothecin analogs and one selected from the group consisting of vinorelbine, vinblastine, vincristine, vindesine, and vinflunine. In some specific embodiments, the second therapeutic agent is a combination of irinotecan and vindesine.

In some embodiments, the second therapeutic agent is a VAC-1 regimen, specifically vincristine, cyclophosphamide, and actinomycin D.

In some embodiments, the second therapeutic agent is a VAC-2 regimen, specifically vincristine, cyclophosphamide, and doxorubicin.

In some embodiments, the second therapeutic agent is a T9 regimen, specifically doxorubicin, methotrexate, cyclophosphamide, actinomycin D, bleomycin, and vincristine.

In some embodiments, the second therapeutic agent is an IE regimen, specifically ifosfamide and etoposide.

In some embodiments, the second therapeutic agent is an AC regimen, specifically doxorubicin and cyclophosphamide.

In some embodiments, the second therapeutic agent is a small molecule targeted anti-tumor drug, including but not limited to protein kinase inhibitors, wherein the protein kinase inhibitors include, but are not limited to, tyrosine kinase inhibitors, serine and/or threonine kinase inhibitors, and poly ADP-ribose polymerase (PARP) inhibitors; targets of the inhibitors include, but are not limited to, epidermal growth factor receptor (EGFR), anaplastic lymphoma (ALK), MET gene, ROS1 gene, HER2 gene, RET gene, BRAF gene, PI3K signaling pathway, discoidin death receptor 2 (DDR2) gene, fibroblast growth factor receptor 1 (FGFR1), neurotrophic tyrosine kinase type 1 receptor (NTRK1) gene, and KRAS gene. Targets of the small molecule targeted anti-tumor drug further include COX-2 (cyclooxygenase-2), APE1 (apurinic apyrimidinic endonuclease ), VEGFR-2 (vascular endothelial growth factor receptor-2), CXCR-4 (chemokine receptor-4), MMP (matrix metalloproteinase), IGF-1R (insulin-like growth factor receptor), Ezrin, PEDF (pigment epithelium derived factor), AS, ES, OPG (osteoprotegerin), Src, IFN, ALCAM (activated leukocyte cell adhesion molecule), HSP, JIP1, GSK-3β (glycogen synthesis kinase 3β), CyclinD1 (cell cycle regulatory protein), CDK4 (cyclin-dependent kinase), TIMP1 (tissue inhibitor of metalloproteinase), THBS3, PTHR1 (parathyroid hormone-related protein receptor 1), TEM7 (tumor endothelial marker 7), COPS3, and cathepsin K. Examples of small molecule targeted anti-tumor drug include, but are not limited to, one or more of erlotinib, afatinib, crizotinib, ceritinib, vemurafenib, dabrafenib, cabozantinib, gefitinib, dacomitinib, osimertinib, alectinib, brigatinib, lorlatinib, trametinib, larotrectinib, icotinib, lapatinib, vandetanib, selumetinib, sorafenib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, rociletinib, nintedanib, lenalidomide, everolimus, LOXO-292, vorolanib, bemcentinib, capmatinib, entrectinib, TAK-931, ALT-803, palbociclib, famitinib L-malate, LTT-462, BLU-667, ningetinib, tipifarnib, poziotinib, DS-1205c, capivasertib, SH-1028, dimethyldiguanide, seliciclib, OSE-2101, APL-101, berzosertib, idelalisib, lerociclib, ceralasertib, PLB-1003, tomivosertib, AST-2818, SKLB-1028, D-0316, LY-3023414, allitinib, MRTX-849, AP-32788, AZD-4205, lifirafenib, vactosertib, mivebresib, napabucasin, sitravatinib, TAS-114, molibresib, CC-223, rivoceranib, CK-101, LXH-254, simotinib, GSK-3368715, TAS-0728, masitinib, tepotinib, HS-10296, AZD-4547, merestinib, olaptesed pegol, galunisertib, ASN-003, gedatolisib, defactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, epitinib succinate, tesevatinib, SPH-1188-11, BPI-15000, copanlisib, niraparib, olaparib, veliparib, talazoparib tosylate, DV-281, siremadlin, telaglenastat, MP-0250, GLG-801, ABTL-0812, bortezomib, panobinostat, tucidinostat, vorinostat, resminostat, epacadostat, tazemetostat, entinostat, mocetinostat, and quisinostat. In some embodiments, the small molecule targeted anti-tumor drug is one or more of sorafenib, everolimus, erlotinib, afatinib, crizotinib, ceritinib, vemurafenib, dabrafenib, cabozantinib, gefitinib, dacomitinib, osimertinib, alectinib, brigatinib, lorlatinib, trametinib, larotrectinib, icotinib, lapatinib, vandetanib, selumetinib, olmutinib,

savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib and nintedanib.

In some embodiments, the administered amount of the compound of formula I or the pharmaceutically acceptable salt thereof in the combination can be determined according to the severity of a disease, the response of the disease, any treatment-related toxicity, and the age and health of a patient. In some embodiments, the daily dose of the compound of formula I or the pharmaceutically acceptable salt thereof is 3 mg to 30 mg. In some embodiments, the daily dose of the compound of formula I or the pharmaceutically acceptable salt thereof is 5 mg to 20 mg. In some embodiments, the daily dose of the compound of formula I or the pharmaceutically acceptable salt thereof is 8 mg to 16 mg. In some embodiments, the daily dose of the compound of formula I or the pharmaceutically acceptable salt thereof is 8 mg to 14 mg. In a specific embodiment, the daily dose of the compound of formula I or the pharmaceutically acceptable salt thereof is 8 mg. In a specific embodiment, the daily dose of the compound of formula I or the pharmaceutically acceptable salt thereof is 10 mg. In a specific embodiment, the daily dose of the compound of formula I or the pharmaceutically acceptable salt thereof is 12 mg.

The compound of formula I or the pharmaceutically acceptable salt thereof can be administered once or multiple times daily. In some embodiments, the compound of formula I or the pharmaceutically acceptable salt thereof is administered once daily.

The dosage regimen of the compound of formula I can be determined comprehensively depending on the activity and toxicity of the drug, tolerance of a patient, etc.

Preferably, the compound of formula I or the pharmaceutically acceptable salt thereof is administered in an intermittent regimen. The intermittent regimen includes treatment periods and interruption periods. In the treatment period, the compound of formula I or the pharmaceutically acceptable salt thereof can be administered once or multiple times daily. For example, the compound of formula I or the pharmaceutically acceptable salt thereof is administered daily in the treatment period, and then the treatment is interrupted during the interruption period, followed by the treatment period and then the interruption period, over and over again. The ratio of the treatment period to the interruption period in days is 2:0.5-2:5, preferably 2:0.5-2:3, more preferably 2:0.5-2:2, and even more preferably 2:0.5-2:1.

In some embodiments, the compound of formula I or the pharmaceutically acceptable salt thereof is administered according to one of the following intermittent regimens: consecutively 2-week treatment and then 2-week interruption, consecutively 2-week treatment and then 1-week interruption, and consecutively 5-day treatment and then 2-day interruption, wherein the cycle can be repeated multiple times.

In some embodiments, the compound of formula I or the pharmaceutically acceptable salt thereof and the second therapeutic agent are administered simultaneously, sequentially, or nonsimultaneously. In some embodiments, the compound of formula I or the pharmaceutically acceptable salt thereof and the second therapeutic agent are administered after being prepared into a pharmaceutical composition.

In some embodiments, the combination for use in treating Ewing's sarcoma includes, but is not limited to, formulations suitable for any of oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations.

The compound of formula I or the pharmaceutically acceptable salt thereof is preferably preparations suitable for oral administration, including tablets, capsules, powders, granules, dripping pills, pastes, pulvis and the like, and preferably tablets and capsules. The tablet may be a common tablet, a dispersible tablet, an effervescent tablet, a sustained-release tablet, a controlled-release tablet or an enteric coated tablet; the capsule may be a common capsule, a sustained-release capsule, a controlled-release capsule or an enteric coated capsule. The oral preparation may be prepared by a conventional method using a pharmaceutically acceptable carrier well known in the art. The pharmaceutically acceptable carrier includes fillers, absorbents, wetting agents, binders, disintegrants, lubricants, and the like. The fillers include starch, lactose, mannitol, microcrystalline cellulose, and the like. The absorbents include calcium sulfate, calcium hydrogen phosphate, calcium carbonate, and the like. The wetting agents include water, ethanol, and the like. The binders include hydroxypropyl methylcellulose, polyvidone, microcrystalline cellulose, and the like. The disintegrants include croscarmellose sodium, crospovidone, surfactants, low-substituted hydroxypropyl cellulose, and the like. The lubricants include magnesium stearate, talcum powder, polyethylene glycol, sodium dodecyl sulfate, colloidal silicon dioxide, talcum powder, and the like. The pharmaceutically acceptable excipient further includes coloring agents, sweeteners and the like.

In certain specific embodiments, irinotecan is administered intravenously at a dosage of 10-40 $mg/m^2$; and meanwhile the compound of formula I or the pharmaceutically acceptable salt thereof is orally administered once or multiple times daily at a dosage selected from but not limited to 3-30 mg, and the administration regimen is consecutively 2-week treatment and then 1-week interruption.

In certain specific embodiments, irinotecan is administered intravenously at a dosage of 10-40 $mg/m^2$, vindesine is administered at a dosage of 1-4$mg/m^2$; and meanwhile the compound of formula I or the pharmaceutically acceptable salt thereof is orally administered once or multiple times daily at a dosage selected from but not limited to 3-30 mg, and the

administration regimen is consecutively 2-week treatment and then 1-week interruption.

In certain specific embodiments, irinotecan is administered at a dosage 10-40 mg/m$^2$, vindesine is administered at a dosage of 1-4 mg/m$^2$, and the compound of formula I or the pharmaceutically acceptable salt thereof is orally administered once or multiple times daily at a dosage of 8-12 mg, while the administration regimen is consecutively 2-week treatment and then 1-week interruption. In some specific embodiments, irinotecan is administered at a dosage of 10, 15, 20, 25, 30, 35 or 40 mg/m$^2$ each time, vindesine is administered at a dosage of 1, 2, 3 or 4 mg/m$^2$ each time, and the compound of formula I or the pharmaceutically acceptable salt thereof is orally administered daily at a dosage of 8-12 mg, while the administration regimen is consecutively 2-week treatment and then 1-week interruption.

In certain specific embodiments, every three weeks constitute a treatment cycle, wherein irinotecan is administered on days 1-5 and 8-10 of each cycle, vindesine is administered on days 1 and 8 of each cycle, and the compound of formula I or the pharmaceutically acceptable salt thereof is administered on days 1-14 of each cycle.

In a specific embodiment, every three weeks constitute a treatment cycle, wherein irinotecan is administered once daily on days 1-5 and 8-10 of each cycle at a dosage of 10, 15 or 20 mg/m$^2$, vindesine is administered once daily on days 1 and 8 of each cycle at a dosage of 2, 3 or 4 mg/m$^2$, and the compound of formula I or the pharmaceutically acceptable salt thereof is administered once daily on days 1-14 of each cycle at a dosage of 8-12 mg.

In certain specific embodiments, vincristine is administered intravenously once a week at a dosage of 1.5 mg/m$^2$, cyclophosphamide is administered intravenously once a week at a dosage of 500 mg/m$^2$, and the treatment with the two chemotherapeutic drugs lasts for 6 weeks; after 6 weeks, the two chemotherapeutic drugs are each administered intravenously once a week at the same dosage described above, and meanwhile the compound of formula I or the pharmaceutically acceptable salt thereof is orally administered once or multiple times daily at a dosage of 3-30 mg, while the administration regimen is consecutively 2-week treatment and then 1-week interruption and radical operation is also performed.

In certain specific embodiments, actinomycin D is administered intravenously at a dosage of 450 μg/m$^2$ (once daily, 5 times in total); intravenous administration of doxorubicin at a dosage of 20 mg/m$^2$ is started on days 15 and 29 ( once daily, 3 times in total for each); intravenous administration of vincristine at a dosage of 1.5 mg/m$^2$ is started on day 43 (once a week, 4 times in total), and cyclophosphamide is administered intravenously at a dosage of 1200 mg/m$^2$ (once every 2 weeks, 2 times in total); the compound of formula I or the pharmaceutically acceptable salt thereof is orally administered once or multiple times daily at a dosage selected from but not limited to 3-30 mg, and the administration regimen is consecutively 2-week treatment and then 1-week interruption.

In certain specific embodiments, the compound of formula I or the pharmaceutically acceptable salt thereof is used in combination with a VAC-1 regimen. The method is as follows: vincristine is administered intravenously at a dosage of 2 mg on day 1; actinomycin D is infused intravenously at a dosage of 2 mg/m$^2$ on day 1; cyclophosphamide is infused intravenously at a dosage of 1200 mg/m$^2$ on day 1; the compound of formula I or the pharmaceutically acceptable salt thereof is orally administered once or multiple times daily at a dosage selected from but not limited to 3-30 mg, and the administration regimen is consecutively 2-week treatment and then 2-week interruption , with 28 days being a cycle.

In certain specific embodiments, the compound of formula I or the pharmaceutically acceptable salt thereof is used in combination with a VAC-2 regimen. The method is as follows: vincristine is administered intravenously at a dosage of 2 mg on day 1; doxorubicin is infused intravenously at a dosage of 75 mg/m$^2$ on day 1; cyclophosphamide is infused intravenously at a dosage of 1200 mg/m$^2$ on day 1; the compound of formula I or the pharmaceutically acceptable salt thereof is orally administered once or multiple times daily at a dosage selected from but not limited to 3-30 mg, and the administration regimen is consecutively 5-day treatment and then 2-day interruption, with 7 days being a cycle.

In certain specific embodiments, the compound of formula I or the pharmaceutically acceptable salt thereof is used in combination with a T9 regimen. The method is as follows: doxorubicin is infused intravenously at a dosage of 20 mg/m$^2$ on days 1-3 and 42-44; methotrexate is infused intravenously at a dosage of 12 mg/m$^2$ on days 1-3 and 42-44; cyclophosphamide is infused intravenously at a dosage of 1.2 g/m$^2$ on days 1 and 42; actinomycin D is infused intravenously at a dosage of 0.5 mg/m$^2$ on days 21-23; bleomycin is infused intravenously at a dosage of 10 mg/m$^2$ on days 21-23; vincristine is infused intravenously at a dosage of 1.5 mg/m$^2$ on days 1, 7, 14, 21 and 30; the compound of formula I or the pharmaceutically acceptable salt thereof is orally administered once or multiple times daily at a dosage selected from but not limited to 3-30 mg, and the administration regimen is consecutively 5-day treatment and then 2-day interruption.

In certain specific embodiments, the compound of formula I or the pharmaceutically acceptable salt thereof is used in combination with an IE regimen and an AC regimen sequentially. The method is as follows: ifosfamide is infused intravenously at a dosage of 1.6 g/m$^2$ on days 1-5; etoposide is infused intravenously at a dosage of 100 mg/m$^2$ on day 15; 21 days constitute one cycle, and after 3 cycles, doxorubicin is infused intravenously at a dosage of 35 mg/m$^2$ on day 8; cyclophosphamide is orally administered at a dosage of 1.50 g/m$^2$ on days 1-7; the compound of formula I or the pharmaceutically acceptable salt thereof is orally administered once or multiple times daily at a dosage selected from but not limited to 3-30 mg, and the administration regimen is consecutively 2-week treatment and then 1-week interruption.

Described herein, but not encompassed by the present invention, is a method for treating Ewing's sarcoma,

which comprises administering to a patient in need thereof, simultaneously, at intervals or sequentially, a therapeutically effective amount of a compound of formula I or a pharmaceutically acceptable salt thereof, and a therapeutically effective amount of a second therapeutic agent.

Described herein, but not encompassed by the present invention, is a combined pharmaceutical composition for use in treating Ewing's sarcoma, which comprises a compound of formula I or a pharmaceutically acceptable salt thereof, and a second therapeutic agent.

Compared with the prior art, the present invention has the following beneficial effects: the compound of formula I or the pharmaceutically acceptable salt thereof is used for treating Ewing's sarcoma, and has remarkable therapeutic effect; the compound of formula I or the pharmaceutically acceptable salt thereof is used in combination with the second therapeutic agent, and the compound of formula I or the pharmaceutically acceptable salt thereof can obviously enhance the killing effect of the drug, particularly the chemotherapeutic drug, on Ewing's sarcoma, and meanwhile reduce the dosage of the chemotherapeutic drug, thereby reducing the side effect. The present invention provides a new idea for treating Ewing's sarcoma, in particular for the second-line treatment of the Ewing's sarcoma which is after failure of a prior treatment with radiotherapy and chemotherapeutic drugs.

Unless otherwise stated, the following terms used in the specification and claims shall have the following meanings for the purposes of the present invention.

In the present application, in the case of anlotinib, the active ingredient always refer to the compound of formula I.

In the present application, unless otherwise stated, the dosages and ranges provided herein for the compound of formula I or the pharmaceutically acceptable salt thereof are based on the molecular weight of the free base of the compound of formula I.

"Patient" refers to a mammal, preferably a human. As used herein, "patient," "subject," or "entity" are used interchangeably.

As used herein, the term "combined pharmaceutical composition" refers to a combination of two or more active ingredients (administered as the respective active ingredients themselves, or as their respective derivatives like pharmaceutically acceptable salts or esters, prodrugs, or compositions) that are administered simultaneously or sequentially. As used herein, the terms "combined pharmaceutical composition", "combination" and "pharmaceutical combination" are used interchangeably.

As used herein, "combined use" or "use in combination" means that two or more active substances may be administered to a subject simultaneously or sequentially in any order as a single formulation.

"Pharmaceutically acceptable" refers to that when a substance is used for preparing a pharmaceutical composition, the pharmaceutical composition is generally safe, non-toxic, and desirable biologically and otherwise, and inclusion of the substance is acceptable for pharmaceutical use in human.

The "pharmaceutically acceptable salt" includes, but is not limited to, acid addition salts of inorganic acids such as hydrochloric acid, hydrobromic acid, sulfuric acid, nitric acid and phosphoric acid, or acid addition salts of organic acids such as acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid, and stearic acid.

The term "therapeutically effective amount" refers to an amount of a compound that, when administered to a human for use in treating a disease, is sufficient to effectively control the disease.

"Treat" or "treatment" or "treating" refers to any administration of a therapeutically effective amount of a compound, and includes:

(1) suppressing a disease in a human experiencing or exhibiting the pathology or symptomatology of the disease (i.e., preventing further pathological and/or symptomatological progression), or

(2) relieving the disease in a human experiencing or exhibiting the pathology or symptomatology of the disease (i.e., reversing the pathology and/or symptomatology).

As used herein, unless otherwise stated, the terms "comprise", "comprises" and "comprising" or equivalents thereof are open-ended statements and mean that elements, components and steps that are not specified may be included in addition to those listed.

## DETAILED DESCRIPTION

The present invention will be further illustrated with reference to the following specific examples and experimental

examples.

**Example 1. 1-[[[4-(4-fluoro-2-methyl-1*H*-indol-5-yl)oxy-6-methoxyquinolin-7-yl]oxy]methyl]cyclopropylamine dihydrochloride (dihydrochloride of the compound of formula I)**

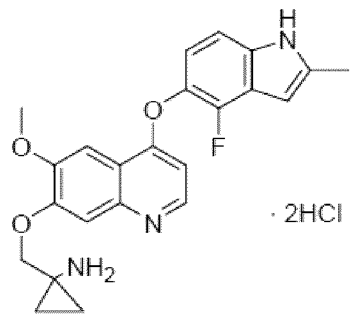

1-[[[4-(4-fluoro-2-methyl-1*H*-indol-5-yl)oxy-6-methoxyquinolin-7-yl]oxy]methyl]cyclopropylamine was prepared by referring to Example 24 of WO2008112407, and then the title compound was prepared by referring to the preparation method of "Examples of salt formation" in the specification of WO2008112407.
Alternatively, the title compound was prepared by referring to the method disclosed in Chinese Patent Application No. CN102344438A.

**Example 2. Preparation of Capsule of 1-[[[4-(4-fluoro-2-methyl-1*H*-indol-5-yl)oxy-6-methoxyquinolin-7-yl] oxy] methyl]cyclopropylamine dihydrochloride (dihydrochloride of the compound of formula I)**

| APIs and excipients | dosage (1000 capsules) |
|---|---|
| Dihydrochloride of the compound of formula I | 14.16 g (equivalent to 12 g of the compound of formula I) |
| Mannitol | 89 g |
| Microcrystalline cellulose | 138.4 g |
| Hydroxypropyl cellulose | 5.9 g |
| Magnesium stearate | 0.99 g |

The dihydrochloride of the compound of formula I was ground, sieved with an 80-mesh sieve, and well mixed with mannitol and hydroxypropyl cellulose. A predetermined amount of microcrystalline cellulose was added, and the resulting mixture was well mixed and sieved with a 0.8-mm sieve. Finally, a predetermined amount of magnesium stearate was added and the resulting mixture was well mixed to fill capsules.
Capsules with different amount of the dihydrochloride of the compound of formula I can be prepared as per the same proportions and formulation as described above.

**Example 3. Efficacy Experiment**

Test drug:

1-[[[4-(4-fluoro-2-methyl-1*H*-indol-5-yl)oxy-6-methoxyquinolin-7-yl]oxy]methyl]cyclopropylamine dihydrochloride (dihydrochloride of the compound of formula I for short); vincristine; doxorubicin; cyclophosphamide.

**1. Effect of dihydrochloride of the compound of formula I on growth of Ewing's sarcoma**

Preparation method:

The dihydrochloride salt of the compound of formula I was dissolved in water to a concentration of 25 mg/mL and then further diluted with distilled water to a concentration of 250 μg/mL.

Preparation of experimental animals:

BALB/cA-nude mice, feeding environment: SPF grade. Nude mice were inoculated subcutaneously with human Ewing's sarcoma cells A673, and randomly grouped (d0) after tumors grew to 100-250 $mm^3$.

Experimental procedures:

The dihydrochloride of the compound of formula I (a dosage of 6.25 mg/kg) was orally administered daily to the animals in a volume of 10 mL/kg for 14 consecutive days. The tumor volume was measured 2-3 times per week, the mice were weighed, and the data was recorded.

The equation for calculating tumor volume (V) is as follows: $V = 1/2 \times a \times b^2$, where a and b represent length and width, respectively.

T/C (%) = (T - T0)/(C - C0) × 100%, where T and C represent the tumor volumes of the administration group mice and the control group (i.e., solvent group) mice, respectively, at the end of the experiment; T0 and C0 represent the tumor volumes of administration group mice and the control group (i.e., solvent group) mice, respectively, at the time of grouping and administration (d0).

The equation for calculating relative tumor volume (RTV) after administration is as follows: RTV = TV/TV0 (TV represents tumor volume at each measurement after administration, and TV0 represents tumor volume at the time of grouping and administration (d0)).

Experimental results show that 1-[[[4-(4-fluoro-2-methyl-1*H*-indol-5-yl)oxy-6-methoxyquinolin-7-yl]oxy]methyl] cyclopropylamine dihydrochloride (dihydrochloride of the compound of formula I) can significantly inhibit the growth of Ewing's sarcoma.

**2. Effect of the combination of the dihydrochloride of the compound of formula I and a second therapeutic agent on proliferation of Ewing's sarcoma cells**

Preparation method:

The test drugs were dissolved in dimethyl sulfoxide to prepare a 100 mmol/L mother solution, which was stored at -20 °C for later use. When in use, the mother solution was prepared with a DMEM serum culture medium to reach a required concentration; vincristine, doxorubicin and cyclophosphamide were mixed (VAC-2 regimen), and the dihydrochloride diluent of the compound of formula I was mixed with vincristine, doxorubicin and cyclophosphamide.

Cell culturing:

Human Ewing's sarcoma cell line A673 was cultured in a DMEM complete culture medium containing 10% fetal bovine serum and 0.1 g/L streptomycin and penicillin (final concentration: 100 $U \cdot mL^{-1}$), and the mixture was incubated in an incubator at 37 °C/5% $CO_2$. When the cell confluence reached about 85%, the cells were mixed and digested using 0.02% EDTA and 0.25% trypsin, and then the cells were collected, centrifuged at 1000 r/min, and subcultured.

Experimental procedures:

The $IC_{50}$ value can be measured by a conventional method in the art (e.g., MTT method), or can be measured by the following method (MTT method):

Cells in logarithmic growth phase were inoculated in a 96-well culture plate (180 μL/well, $10^5$ cells/well); after 2 h of incubation at 37 °C/5% $CO_2$, the cells were added with the dihydrochloride of the compound of formula I (solutions at gradient concentrations of 0, 0.005, 0.1, 0.05, 0.1, 0.5, 1.5, 4, 12 and 30 μg/mL), 2 μg/mL vincristine, 75 μg/mL doxorubicin and 500 μg/mL cyclophosphamide, and the resulting mixtures were incubated. Two duplicate wells were set for each concentration, 20 μL added for each well. Meanwhile, wells for normal saline solvent controls with corresponding concentrations and cell-free zeroing wells were set. The tumor cells were incubated at 37 °C/5% $CO_2$ for 24 h (i.e., 48 h in total); after the drug effect was finished, MTT working solution was added into each well, and after 4 h, the combination solution of three drugs was added for dissolving. The resulting mixtures were left to stand overnight at 37 °C. The next day, OD values were measured at 570 nm and 630 nm wavelengths using a microplate reader (SPECTRA max 190) (calculated by subtracting OD values measured at 630 nm (the control wavelength) from all OD values measured at 570 nm), and the cell growth inhibition rate was calculated according to the following equation:

$$\text{Inhibition rate} = (\text{OD value of control well} - \text{OD value of administration well})/\text{OD value of control well} \times 100\%$$

Half maximal inhibitory concentration $IC_{50}$ was calculated according to inhibition rates at all concentrations using GraphPad Prism 5 software.

Experimental results:

The *in vitro* pharmacodynamic effect of the combined therapy (dihydrochloride of the compound of formula I, vincristine, doxorubicin and cyclophosphamide) and the positive control (vincristine, doxorubicin and cyclophosphamide) on human Ewing's sarcoma A673 cells shows that the combination of the dihydrochloride of the compound of formula I with a second therapeutic agent has definite inhibition effect on the proliferation of the human Ewing's sarcoma A673 cells.

**3. Effect of the combination of the dihydrochloride of the compound of formula I and a second therapeutic agent on apoptosis of Ewing's sarcoma cells**

Detection of apoptosis using flow cytometry

Human Ewing's sarcoma A673 cells in logarithmic growth phase were added at $10^5$ cells/well to a DMEM complete culture medium containing 2 μg/mL vincristine, 75 μg/mL doxorubicin and 500 μg/mL cyclophosphamide, and to a DMEM complete culture medium containing the dihydrochloride of the compound of formula I (at concentrations of 0, 0.005, 0.1, 0.05, 0.1, 0.5, 1.5, 4, 12 and 30 μg/mL), 2 μg/mL vincristine, 75 μg/mL doxorubicin and 500 μg/mL cyclophosphamide. The resulting mixtures were incubated for 24 h, and then the cells were collected, centrifuged at 1000 r/min for 3-5 min, and washed with PBS. Then the apoptosis of the cells was detected using an Annexin-V-FITC/PI apoptosis detection kit. The cells were resuspended in 100 μL of 1× Binding buffer solution, and 5 μL of Annexin-V-FITC and 2.5 μL of PI dye were added. The mixture was shaken and mixed well in the absence of light, reacted for 15 min at room temperature, added with 300 μL of 1× Binding buffer solution, mixed well, and then subjected to detection by a flow cytometer. The test was repeated 3 times.

Test results:

The results based on a classical Annexin-V-FITC/PI apoptosis detection method show that the dihydrochloride of the compound of formula I at different concentrations can remarkably enhance apoptosis of an Ewing's sarcoma cell line caused by the vincristine + doxorubicin + cyclophosphamide regimen (VAC-2 regimen).

**Example 4. Clinical Trial**

A clinical trial was carried out in Ewing's sarcoma patients with measurable lesions (according to RECIST 1.1) for the combination of the dihydrochloride capsule of the compound of formula I and a second therapeutic agent (VAC-2 regimen). Patients in the patient group comprised patients who had previously received chemotherapy and those who had not. The patients were orally administered with 12 mg of the dihydrochloride capsule of the compound of formula I once daily (2 weeks of treatment and 1 week of interruption constitute one treatment cycle), and meanwhile the patients were treated with the VAC-2 regimen: intravenous infusion of 2 mg of vincristine on day 1, intravenous infusion of 75 $mg/m^2$ of doxorubicin on day 1, and intravenous infusion of 1200 $mg/m^2$ of cyclophosphamide on day 1 (28 days constitute one cycle). The evaluation endpoints included: efficacy endpoints, e.g., progression-free survival (PFS), objective response rate (ORR), duration of response (DOR), stable disease (SD) rate, clinical benefit rate (CBR), overall survival (OS), etc.; safety endpoints, e.g., incidence and severity of adverse events; and quality of life. The clinical trial results are as follows: The combination of the dihydrochloride of the compound of formula I and the VAC-2 regimen is effective for treatment of Ewing's sarcoma, and can prolong overall survival, etc.

**Example 5**

Patient, male, 29 years old, left pubic aspiration biopsy performed in November 2014, pathologically diagnosed as small round cell malignancy. Clinical diagnosis: metastasis to both lungs following left pubic Ewing's sarcoma postoperative chemotherapy.

From November 2014 to October 2018, the patient had received 9 cycles of treatment with vincristine (VCR), doxorubicin (ADM) and cyclophosphamide (CTX), and 10 cycles of treatment with ifosfamide (IFO) and etoposide (VP-16); grade III myelosuppression was observed a plurality of times during chemotherapy. In March 2015, the patient received "huge pelvic tumor resection + hemipelvectomy under exclusion of hip joint" under general anesthesia. Postoperative pathology: small cell malignancy (left hip), which was most probably Ewing's sarcoma according to immunophenotyping and molecular detection results. The patient then received 2 cycles of chemotherapy with "vincristine

+ doxorubicin + cyclophosphamide" from December 2018, and the CT scanning and other examinations in March 2019 showed disease progression in lungs, and the chemotherapy effect is not ideal. The patient then started the treatment with an anlotinib dihydrochloride capsule (the active ingredient is the compound of formula I), which was orally administered once daily at a dosage of 12 mg (2 weeks of treatment and 1 week of interruption constitute one treatment cycle).

One week prior to treatment with the anlotinib dihydrochloride capsule, CT scan showed that the sum of the diameters of measurable target lesions (nodules at left lower lung) was 16 mm. CT scanning was carried out periodically after administration, and the sum of the diameters of the target lesions was reduced to 10 mm after 10 weeks of treatment. The therapeutic effect featuring reduce of 37.5% was evaluated as partial response (PR). The sum of the diameters of the target lesions was reduced to 9 mm after 16 treatment cycles, the therapeutic effect was evaluated as PR, and no new lesions were observed. During treatment, the overall tolerability was good and the patient is still receiving the treatment.

### Example 6

Patient, male, 28 years old. Aspiration biopsy was performed in 2016 and the pathological diagnosis result was peripheral primitive neuroectodermal tumor. Clinical diagnosis: metastasis to both lungs following sacral Ewing's sarcoma/primitive neuroectodermal tumor postoperative chemotherapy.

From May 2016 to July 2016, the patient received the following treatments successively: liposomal doxorubicin 40 mg d1 + cyclophosphamide 1g d1; ifosfamide 4g d1-3 + etoposide 100 mg d1-3; liposomal doxorubicin 40 mg d1 + cyclophosphamide 1g d1; ifosfamide 4g d1-3 + etoposide 100 mg d1-3. In August 2016, the patient received "posterior sacral tumor resection and fixation" under general anesthesia, and postoperative pathology indicated small round cell malignancy of sacrum, which tends to be Ewing's sarcoma/primitive neuroectodermal tumor. Five cycles of postoperative chemotherapy were carried out, including: epirubicin 110 mg d1 + cyclophosphamide 1 g d1; ifosfamide 4 g d1-3 + etoposide 100 mg d1-3; epirubicin 110 mg d1 + cyclophosphamide 1 g d1 + vindesine 3 mg d1; ifosfamide 4 g d1-3 + etoposide 100 mg d1-3; and epirubicin 110 mg d1 + cyclophosphamide 1 g d1 + vindesine 3 mg d1. After chemotherapy, grade II myelosuppression was observed, and the time of the last chemotherapy was February 2017. In August 2017, the patient was administered successively with irinotecan 40 mg d1-4 + temozolomide 140 mg d1-5, and irinotecan 40 mg. From September 2017, the patient was administered successively with irinotecan 40 mg d1-4 + temozolomide 140 mg d1-5, irinotecan 40 mg, irinotecan 40 mg d1-4 + cyclophosphamide 500 mg d1-4, and irinotecan 40 mg d1-4 + temozolomide 140 mg d1-5.

In January 2019, the patient started the treatment with an anlotinib dihydrochloride capsule, which was orally administered once daily at a dosage of 12 mg (2 weeks of treatment and 1 week of interruption constitute one treatment cycle).

Three days before administration of anlotinib dihydrochloride capsule, CT scan showed that the sum of diameters of measurable target lesions was 36 mm (36 mm at lower lobe of right lung). After each treatment cycle was completed, CT scan was performed immediately, and the results are shown in Table 1 below. The subject has received 20 cycles of drug administration at present, and the doctor, after assessment, advises that the drug administration shall be continued. The overall tolerance was good during the treatment.

Table 1

| Treatment cycle | Sum of diameters of target lesions | Evaluation of therapeutic effect |
|---|---|---|
| Second cycle | 20 mm | PR |
| Sixth cycle | 19.3 mm | PR |
| Eighth cycle | 19 mm | PR |
| Tenth cycle | 19.6 mm | PR |
| Twelfth cycle | 18.6 mm | PR |
| Fourteenth cycle | 16.8 mm | PR |
| Sixteenth cycle | 18.5 mm | PR |
| Twentieth cycle | 18.5 mm | PR |

### Example 7

Patients with advanced Ewing's sarcoma who failed a prior treatment were divided into groups A and B. Group A had 23 patients aged 16 years or over, and they were provided with the following treatment: once-daily oral administration of anlotinib dihydrochloride capsule 12 mg d1-d14 (2 weeks of treatment and 1 week of interruption constitute one

treatment cycle) + once-daily intravenous administration of irinotecan 15 mg/$m^2$ d1-d5, d8-d10 (3 weeks constitute one treatment cycle) + vindesine 3 mg/$m^2$ (administration on d1 and d8, 3 weeks constitute one treatment cycle). Group B had 12 patients younger than 16 years, and they were provided with the following treatment: once-daily oral administration of anlotinib dihydrochloride capsule (2 weeks of treatment and 1 week of interruption constitute one treatment cycle, and the dosages were determined as per body surface area (BSA) and are specifically as follows: BSA < 1.0 $m^2$, 8 mg, d1-d14; BSA ≧ 1.0 $m^2$, 12 mg, d1-d14) + once-daily intravenous administration of irinotecan 20 mg/$m^2$ d1-d5, d8-d10 (3 weeks constitute one treatment cycle) + vindesine 3 mg/$m^2$ d1, d8 (3 weeks constitute one treatment cycle).

The research results of groups A and B were evaluated at week 12: for the two groups, the ORRs at week 12 are 62.5% and 83.3%, respectively, and complete response (CR) is observed in one case of 23 patients aged 16 or over.

## Claims

1. A compound of formula I or a pharmaceutically acceptable salt thereof for use in treating Ewing's sarcoma,

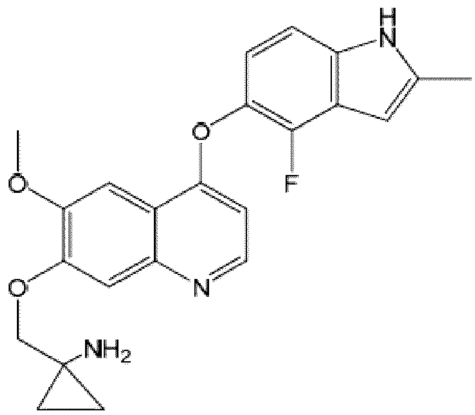

formula I.

2. The compound of formula I or a pharmaceutically acceptable salt thereof for use according to claim 1, wherein the Ewing's sarcoma is endoskeletal Ewing's sarcoma, extraskeletal Ewing's sarcoma or periosteal Ewing's sarcoma; or

   the Ewing's sarcoma is osteolytic Ewing's sarcoma, sclerosing Ewing's sarcoma or hybrid Ewing's sarcoma; or the Ewing's sarcoma is one that is after failure of a prior treatment; preferably, the Ewing's sarcoma is one that is after failure of treatment with radiotherapy and/or a chemotherapeutic drug.

3. The compound of formula I or a pharmaceutically acceptable salt thereof for use according to claim 1 or 2, wherein the pharmaceutically acceptable salt of the compound of formula I is a salt formed by the compound of formula I and any of the following acids: hydrochloric acid, hydrobromic acid, sulfuric acid, nitric acid, phosphoric acid, acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid, and stearic acid; preferably, the pharmaceutically acceptable salt is a hydrochloride or maleate; more preferably, the pharmaceutically acceptable salt is a dihydrochloride.

4. The compound of formula I or a pharmaceutically acceptable salt thereof for use according to any one of claims 1-3, wherein the compound of formula I or the pharmaceutically acceptable salt thereof is administered at a daily dosage of 3 mg to 30 mg, preferably 5 mg to 20 mg, more preferably 8 mg to 16 mg, further preferably 8 mg to 14 mg, and most preferably 8 mg, 10 mg, or 12 mg;
   preferably, the compound of formula I or the pharmaceutically acceptable salt thereof is administered in an intermittent regimen of alternate treatment and interruption periods; preferably, the ratio of the treatment period to the interruption period in days is preferably 2:0.5-2:5, more preferably 2:0.5-2:3, further preferably 2:0.5-2:2, and further more preferably 2:0.5-2:1; still further more preferably, the intermittent regimen is one of the following cycles: consecutively 2-week treatment and then 2-week interruption, consecutively 2-week treatment and then 1-week interruption, and

consecutively 5-day treatment and then 2-day interruption, wherein the cycle is repeated multiple times.

5. The compound of formula I or a pharmaceutically acceptable salt thereof for use according to any one of claims 1-4, wherein the compound of formula I or a pharmaceutically acceptable salt thereof for use in treating Ewing's sarcoma is a preparation suitable for any of oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations.

6. The compound of formula I or a pharmaceutically acceptable salt thereof for use according to any one of claims 1-5, wherein the compound of formula I or a pharmaceutically acceptable salt thereof is in a form of a combined pharmaceutical composition for use in treating Ewing's sarcoma, comprising a compound of formula I or a pharmaceutically acceptable salt thereof and a second therapeutic agent.

7. The compound of formula I or a pharmaceutically acceptable salt thereof for use according to claim 6, wherein the second therapeutic agent is a chemotherapeutic drug and/or a small molecule targeted anti-tumor drug;

preferably, the chemotherapeutic drug is one or more of an alkylating agent, a podophyllum, a camptothecin analog, a taxane, an antimetabolite and an anti-tumor antibiotic;
more preferably, the chemotherapeutic drug is one or more of a platinum-based drug, a fluoropyrimidine derivative, a taxane, a camptothecin analog, a vinca alkaloid, pemetrexed, carmustine, etoposide, teniposide, mitomycin, ifosfamide, cyclophosphamide, azacitidine, pirarubicin, amrubicin, methotrexate, bendamustine, epirubicin, doxorubicin, actinomycin D, dactinomycin, bleomycin, temozolomide, LCL-161, KML-001, sapacitabine, plinabulin, treosulfan, tipiracil hydrochloride, $^{153}$Sm-EDTMP, tegafur-gimeracil-oteracil potassium, and encequidar;
further preferably, the platinum-based drug is one or more of oxaliplatin, cisplatin, carboplatin, nedaplatin and dicycloplatin; the fluoropyrimidine derivative is one or more of gemcitabine, capecitabine, fluorouracil, difuradin, doxifluridine, tegafur, carmofur and trifluridine; the taxane is one or more of paclitaxel, albumin-bound paclitaxel and docetaxel; the camptothecin analog is one or more of camptothecin, hydroxycamptothecin, irinotecan and topotecan; the vinca alkaloid is one or more of vinorelbine, vinblastine, vincristine, vindesine and vinflunine;
preferably, the small molecule targeted anti-tumor drug is a protein kinase inhibitor;
more preferably, the small molecule targeted anti-tumor drug is a tyrosine kinase inhibitor and a serine and/or threonine kinase inhibitor;
further preferably, the small molecule targeted anti-tumor drug is one or more of erlotinib, afatinib, crizotinib, ceritinib, vemurafenib, dabrafenib, cabozantinib, gefitinib, dacomitinib, osimertinib, alectinib, brigatinib, lorlatinib, trametinib, larotrectinib, icotinib, lapatinib, vandetanib, selumetinib, sorafenib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, rociletinib, nintedanib, lenalidomide, everolimus, LOXO-292, vorolanib, bemcentinib, capmatinib, entrectinib, TAK-931, ALT-803, palbociclib, famitinib L-malate, LTT-462, BLU-667, ningetinib, tipifarnib, poziotinib, DS-1205c, capivasertib, SH-1028, dimethyldiguanide, seliciclib, OSE-2101, APL-101, berzosertib, idelalisib, lerociclib, ceralasertib, PLB-1003, tomivosertib, AST-2818, SKLB-1028, D-0316, LY-3023414, allitinib, MRTX-849, AP-32788, AZD-4205, lifirafenib, vactosertib, mivebresib, napabucasin, sitravatinib, TAS-114, molibresib, CC-223, rivoceranib, CK-101, LXH-254, simotinib, GSK-3368715, TAS-0728, masitinib, tepotinib, HS-10296, AZD-4547, merestinib, olaptesed pegol, galunisertib, ASN-003, gedatolisib, defactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, epitinib succinate, tesevatinib, SPH-1188-11, BPI-15000, copanlisib, niraparib, olaparib, veliparib, talazoparib tosylate, DV-281, siremadlin, telaglenastat, MP-0250, GLG-801, ABTL-0812, bortezomib, panobinostat, tucidinostat, vorinostat, resminostat, epacadostat, tazemetostat, entinostat, mocetinostat, and quisinostat;
still further preferably, the small molecule targeted anti-tumor drug is one or more of sorafenib, everolimus, erlotinib, afatinib, crizotinib, ceritinib, vemurafenib, dabrafenib, cabozantinib, gefitinib, dacomitinib, osimertinib, alectinib, brigatinib, lorlatinib, trametinib, larotrectinib, icotinib, lapatinib, vandetanib, selumetinib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib and nintedanib.

8. The compound of formula I or a pharmaceutically acceptable salt thereof for use according to any one of claims 6-7, wherein the second therapeutic agent is a combination of vincristine, cyclophosphamide, and actinomycin D; or

the second therapeutic agent is a combination of vincristine, cyclophosphamide, and doxorubicin; or

the second therapeutic agent is a combination of doxorubicin, methotrexate, cyclophosphamide, actinomycin D, bleomycin, and vincristine; or
the second therapeutic agent is a combination of vincristine, cyclophosphamide, actinomycin D, and/or doxorubicin; or
the second therapeutic agent is a combination of ifosfamide and etoposide; or
the second therapeutic agent is a combination of doxorubicin and cyclophosphamide; or
the second therapeutic agent is a combination of vincristine and cyclophosphamide.

**9.** The compound of formula I or a pharmaceutically acceptable salt thereof for use according to any one of claims 6-8, wherein the second therapeutic agent is one of a VAC regimen, a T9 regimen, an IE regimen and an AC regimen.

**10.** The compound of formula I or a pharmaceutically acceptable salt thereof for use according to any one of claims 6-9, wherein in the combined pharmaceutical composition, the pharmaceutically acceptable salt is a hydrochloride or maleate; more preferably, the pharmaceutically acceptable salt is a dihydrochloride.

**11.** The compound of formula I or a pharmaceutically acceptable salt thereof for use according to any one of claims 6-10, wherein the compound of formula I or the pharmaceutically acceptable salt thereof is administered at a daily dosage of 3 mg to 30 mg;
preferably, the compound of formula I or the pharmaceutically acceptable salt thereof is administered in an intermittent regimen of alternate treatment and interruption periods; preferably, the ratio of the treatment period to the interruption period in days is preferably 2:0.5-2:5, more preferably 2:0.5-2:3, further preferably 2:0.5-2:2, and further more preferably 2:0.5-2:1; still further more preferably, the intermittent regimen is one of the following cycles: consecutively 2-week treatment and then 2-week interruption, consecutively 2-week treatment and then 1-week interruption, and consecutively 5-day treatment and then 2-day interruption, wherein the cycle is repeated multiple times.

**12.** The compound of formula I or a pharmaceutically acceptable salt thereof for use according to any one of claims 6-11, wherein the compound of formula I or the pharmaceutically acceptable salt thereof and the second therapeutic agent are administered simultaneously, sequentially, or nonsimultaneously.

**13.** The compound of formula I or a pharmaceutically acceptable salt thereof for use according to any one of claims 6-12, wherein the combined pharmaceutical composition for use in treating Ewing's sarcoma is a preparation suitable for any of oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations.

**14.** A pharmaceutical composition for use in treating Ewing's sarcoma, comprising a compound of formula I or a pharmaceutically acceptable salt thereof

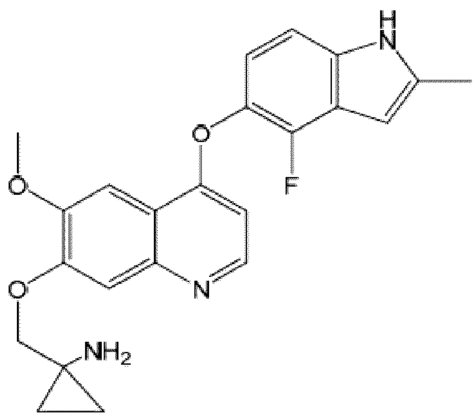

formula I.

**15.** The pharmaceutical composition for use according to claim 14, wherein the Ewing's sarcoma is endoskeletal Ewing's sarcoma, extraskeletal Ewing's sarcoma or periosteal Ewing's sarcoma; or

the Ewing's sarcoma is osteolytic Ewing's sarcoma, sclerosing Ewing's sarcoma or hybrid Ewing's sarcoma; or

the Ewing's sarcoma is one that is after failure of a prior treatment; preferably, the Ewing's sarcoma is one that is after failure of treatment with radiotherapy and/or a chemotherapeutic drug.

## Patentansprüche

1. Verbindung der Formel I oder pharmazeutisch annehmbares Salz davon zur Verwendung bei der Behandlung des Ewing-Sarkoms,

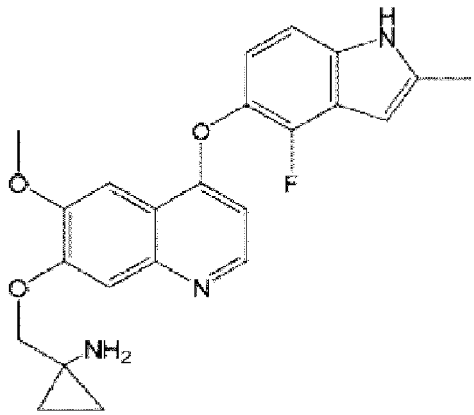

Formel I.

2. Verbindung der Formel I oder pharmazeutisch annehmbares Salz davon zur Verwendung nach Anspruch 1, wobei das Ewing-Sarkom endoskelettales Ewing-Sarkom, extraskelettales Ewing-Sarkom oder periostales Ewing-Sarkom ist; oder

   das Ewing-Sarkom osteolytisches Ewing-Sarkom, sklerosierendes Ewing-Sarkom oder hybrides Ewing-Sarkom ist; oder
   das Ewing-Sarkom eines ist, das nach Versagen einer vorherigen Behandlung vorliegt; bevorzugt, das Ewing-Sarkom eines ist, das nach Versagen einer Behandlung mit Strahlentherapie und/oder einem Chemotherapeutikum vorliegt.

3. Verbindung der Formel I oder pharmazeutisch annehmbares Salz davon zur Verwendung nach Anspruch 1 oder 2, wobei das pharmazeutisch annehmbare Salz der Verbindung der Formel I ein Salz ist, das durch die Verbindung der Formel I und eine beliebige der folgenden Säuren gebildet wird: Salzsäure, Bromwasserstoffsäure, Schwefelsäure, Salpetersäure, Phosphorsäure, Essigsäure, Trifluoressigsäure, Propionsäure, Hexansäure, Heptansäure, Cyclopentanpropionsäure, Glycolsäure, Brenztraubensäure, Milchsäure, Malonsäure, Bernsteinsäure, Apfelsäure, Maleinsäure, Fumarsäure, Weinsäure, Zitronensäure, Benzoesäure, Zimtsäure, Mandelsäure, Methansulfonsäure, Ethansulfonsäure, 1,2-Ethandisulfonsäure, 2-Hydroxyethansulfonsäure, Benzolsulfonsäure, *p*-Chlorbenzolsulfonsäure, *p*-Toluolsulfonsäure, 3-Phenylpropionsäure, Trimethylessigsäure, *t*-Butylessigsäure, Dodecylschwefelsäure, Gluconsäure, Glutaminsäure, Hydroxynaphthoesäure, Salicylsäure und Stearinsäure; bevorzugt, das pharmazeutisch annehmbare Salz ein Hydrochlorid oder Maleat ist; bevorzugter, das pharmazeutisch annehmbare Salz ein Dihydrochlorid ist.

4. Verbindung der Formel I oder pharmazeutisch annehmbares Salz davon zur Verwendung nach einem der Ansprüche 1-3, wobei die Verbindung der Formel I oder das pharmazeutisch annehmbare Salz davon in einer Tagesdosis von 3 mg bis 30 mg, bevorzugt 5 mg bis 20 mg, bevorzugter 8 mg bis 16 mg, weiter bevorzugt 8 mg bis 14 mg und am bevorzugtesten 8 mg, 10 mg oder 12 mg verabreicht wird;
   bevorzugt, die Verbindung der Formel I oder das pharmazeutisch annehmbare Salz davon in einem intermittierenden Regime aus abwechselnden Behandlungs- und Unterbrechungszeiträumen verabreicht wird; bevorzugt, das Verhältnis des Behandlungszeitraums zum Unterbrechungszeitraum in Tagen bevorzugt 2:0,5-2:5, bevorzugter 2:0,5-2:3, weiter bevorzugt 2:0,5-2:2 und weiter bevorzugter 2:0,5-2:1 ist; noch weiter bevorzugt, das intermittierende Regime einer der folgenden Zyklen ist: konsekutive 2-wöchige Behandlung und dann 2-wöchige Unterbrechung, konsekutive 2-wöchige Behandlung und dann 1-wöchige Unterbrechung und konsekutive 5-tägige Behandlung und dann 2-tägige Unterbrechung, wobei der Zyklus mehrfach wiederholt wird.

5. Verbindung der Formel I oder pharmazeutisch annehmbares Salz davon zur Verwendung nach einem der Ansprüche 1-4, wobei die Verbindung der Formel I oder das pharmazeutisch annehmbare Salz davon zur Verwendung bei der Behandlung des Ewing-Sarkoms eine Zubereitung ist, die für beliebige von oralen, parenteralen, intraperitonealen, intravenösen, intraarteriellen, transdermalen, sublingualen, intramuskulären, rektalen, transbukkalen, intranasalen, inhalativen, vaginalen, intraokularen, topischen, subkutanen, intraadipösen, intraartikulären und intrathekalen Verabreichungen geeignet ist.

6. Verbindung der Formel I oder pharmazeutisch annehmbares Salz davon zur Verwendung nach einem der Ansprüche 1-5, wobei die Verbindung der Formel I oder ein pharmazeutisch annehmbares Salz davon in Form einer kombinierten pharmazeutischen Zusammensetzung zur Verwendung bei der Behandlung des Ewing-Sarkoms, umfassend eine Verbindung der Formel I oder ein pharmazeutisch annehmbares Salz davon und ein zweites Therapeutikum, vorliegt.

7. Verbindung der Formel I oder pharmazeutisch annehmbares Salz davon zur Verwendung nach Anspruch 6, wobei das zweite Therapeutikum ein Chemotherapeutikum und/oder ein auf kleine Moleküle gerichtetes Antitumor-Arzneimittel ist;

bevorzugt, das Chemotherapeutikum eines oder mehrere von einem Alkylierungsmittel, einem Podophyllum, einem Camptothecin-Analogon, einem Taxan, einem Antimetaboliten und einem Antitumor-Antibiotikum ist;
bevorzugter, das Chemotherapeutikum eines oder mehrere von einem platinbasierten Arzneimittel, einem Fluorpyrimidin-Derivat, einem Taxan, einem Camptothecin-Analogon, einem Vincaalkaloid, Pemetrexed, Carmustin, Etoposid, Teniposid, Mitomycin, Ifosfamid, Cyclophosphamid, Azacitidin, Pirarubicin, Amrubicin, Methotrexat, Bendamustin, Epirubicin, Doxorubicin, Actinomycin D, Dactinomycin, Bleomycin, Temozolomid, LCL-161, KML-001, Sapacitabin, Plinabulin, Treosulfan, Tipiracil-Hydrochlorid, $^{153}$Sm-EDTMP, Tegafur-Gimeracil-Oteracil-Kalium und Encequidar ist;
weiter bevorzugt, das platinbasierte Arzneimittel eines oder mehrere von Oxaliplatin, Cisplatin, Carboplatin, Nedaplatin und Dicycloplatin ist; das Fluoropyrimidin-Derivat eines oder mehrere von Gemcitabin, Capecitabin, Fluoruracil, Difuradin, Doxifluridin, Tegafur, Carmofur und Trifluridin ist; das Taxan eines oder mehrere von Paclitaxel, albumingebundenem Paclitaxel und Docetaxel ist; das Camptothecin-Analogon ist eines oder mehrere von Camptothecin, Hydroxycamptothecin, Irinotecan und Topotecan ist; das Vincaalkaloid eines oder mehrere von Vinorelbin, Vinblastin, Vincristin, Vindesin und Vinflunin ist;
bevorzugt, das auf kleine Moleküle gerichtete Antitumor-Arzneimittel ein Proteinkinase-Inhibitor ist;
bevorzugter, das auf kleine Moleküle gerichtete Antitumor-Arzneimittel ein Tyrosinkinase-Inhibitor und ein Serin- und/oder Threoninkinase-Inhibitor ist;
weiter bevorzugt, das auf kleine Moleküle gerichtete Antitumor-Arzneimittel eines oder mehrere von Erlotinib, Afatinib, Crizotinib, Ceritinib, Vemurafenib, Dabrafenib, Cabozantinib, Gefitinib, Dacomitinib, Osimertinib, Alectinib, Brigatinib, Lorlatinib, Trametinib, Larotrectinib, Icotinib, Lapatinib, Vandetanib, Selumetinib, Sorafenib, Ohnutinib, Savolitinib, Fruquintinib, Entrectinib, Dasatinib, Ensartinib, Lenvatinib, Itacitinib, Pyrotinib, Binimetinib, Erdafitinib, Axitinib, Neratinib, Cobimetinib, Acalabrutinib, Famitinib, Masitinib, Ibrutinib, Rociletinib, Nintedanib, Lenalidomid, Everolimus, LOXO-292, Vorolanib, Bemcentinib, Capmatinib, Entrectinib, TAK-931, ALT-803, Palbociclib, Famitinib-L-Malat, LTT-462, BLU-667, Ningetinib, Tipifarnib, Poziotinib, DS-1205c, Capivasertib, SH-1028, Dimethyldiguanid, Seliciclib, OSE-2101, APL-101, Berzosertib, Idelalisib, Lerociclib, Ceralasertib, PLB-1003, Tomivosertib, AST-2818, SKLB-1028, D-0316, LY-3023414, Allitinib, MRTX-849, AP-32788, AZD-4205, Lifirafenib, Vactosertib, Mivebresib, Napabucasin, Sitravatinib, TAS-114, Molibresib, CC-223, Rivoceranib, CK-101, LXH-254, Simotinib, GSK-3368715, TAS-0728, Masitinib, Tepotinib, HS-10296, AZD-4547, Merestinib, Olaptesed Pegol, Galunisertib, ASN-003, Gedatolisib, Defactinib, Lazertinib, CKI-27, S-49076, BPI-9016M, RF-A-089, RMC-4630, AZD-3759, Antroquinonol, SAF-189s, AT-101, TTI-101, Naputinib, LNP-3794, HH-SCC-244, ASK-120067, CT-707, Epitinib-Succinat, Tesevatinib, SPH-1188-11, BPI-15000, Copanlisib, Niraparib, Olaparib, Veliparib, Talazoparib-Tosylat, DV-281, Siremadlin, Telaglenastat, MP-0250, GLG-801, ABTL-0812, Bortezomib, Panobinostat, Tucidinostat, Vorinostat, Resminostat, Epacadostat, Tazemetostat, Entinostat, Mocetinostat und Quisinostat ist;
noch weiter bevorzugt das auf kleine Moleküle gerichtete Antitumor-Arzneimittel eines oder mehrere von Sorafenib, Everolimus, Erlotinib, Afatinib, Crizotinib, Ceritinib, Vemurafenib, Dabrafenib, Cabozantinib, Gefitinib, Dacomitinib, Osimertinib, Alectinib, Brigatinib, Lorlatinib, Trametinib, Larotrectinib, Icotinib, Lapatinib, Vandetanib, Selumetinib, Olmutinib, Savolitinib, Fruquintinib, Entrectinib, Dasatinib, Ensartinib, Lenvatinib, Itacitinib, Pyrotinib, Binimetinib, Erdafitinib, Axitinib, Neratinib, Cobimetinib, Acalabrutinib, Famitinib, Masitinib, Ibrutinib und Nintedanib ist.

8. Verbindung der Formel I oder pharmazeutisch annehmbares Salz davon zur Verwendung nach einem der Ansprüche

6-7, wobei das zweite Therapeutikum eine Kombination aus Vincristin, Cyclophosphamid und Actinomycin D ist; oder

das zweite Therapeutikum eine Kombination aus Vincristin, Cyclophosphamid und Doxorubicin ist; oder
das zweite Therapeutikum eine Kombination aus Doxorubicin, Methotrexat, Cyclophosphamid, Actinomycin D, Bleomycin und Vincristin ist; oder
das zweite Therapeutikum eine Kombination aus Vincristin, Cyclophosphamid, Actinomycin D und/oder Doxorubicin ist; oder
das zweite Therapeutikum eine Kombination aus Ifosfamid und Etoposid ist; oder
das zweite Therapeutikum eine Kombination aus Doxorubicin und Cyclophosphamid ist; oder
das zweite Therapeutikum eine Kombination aus Vincristin und Cyclophosphamid ist.

**9.** Verbindung der Formel I oder pharmazeutisch annehmbares Salz davon zur Verwendung nach einem der Ansprüche 6-8, wobei das zweite Therapeutikum eines aus einem VAC-Regime, einem T9-Regime, einem IE-Regime und einem AC-Regime ist.

**10.** Verbindung der Formel I oder pharmazeutisch annehmbares Salz davon zur Verwendung nach einem der Ansprüche 6-9, wobei, in der kombinierten pharmazeutischen Zusammensetzung, das pharmazeutisch annehmbare Salz ein Hydrochlorid oder Maleat ist; bevorzugter, das pharmazeutisch annehmbare Salz ein Dihydrochlorid ist.

**11.** Verbindung der Formel I oder pharmazeutisch annehmbares Salz davon zur Verwendung nach einem der Ansprüche 6-10, wobei die Verbindung der Formel I oder das pharmazeutisch annehmbare Salz davon in einer Tagesdosis von 3 mg bis 30 mg verabreicht wird;
bevorzugt, die Verbindung der Formel I oder das pharmazeutisch annehmbare Salz davon in einem intermittierenden Regime aus abwechselnden Behandlungs- und Unterbrechungszeiträumen verabreicht wird; bevorzugt, das Verhältnis des Behandlungszeitraums zum Unterbrechungszeitraum in Tagen bevorzugt 2:0,5-2:5, bevorzugter 2:0,5-2:3, weiter bevorzugt 2:0,5-2:2 und weiter bevorzugter 2:0,5-2:1 ist; noch weiter bevorzugt, das intermittierende Regime einer der folgenden Zyklen ist: konsekutive 2-wöchige Behandlung und dann 2-wöchige Unterbrechung, konsekutive 2-wöchige Behandlung und dann 1-wöchige Unterbrechung und konsekutive 5-tägige Behandlung und dann 2-tägige Unterbrechung, wobei der Zyklus mehrfach wiederholt wird.

**12.** Verbindung der Formel I oder pharmazeutisch annehmbares Salz davon zur Verwendung nach einem der Ansprüche 6-11, wobei die Verbindung der Formel I oder das pharmazeutisch annehmbare Salz davon und das zweite Therapeutikum gleichzeitig, sequenziell oder nicht gleichzeitig verabreicht werden.

**13.** Verbindung der Formel I oder pharmazeutisch annehmbares Salz davon zur Verwendung nach einem der Ansprüche 6-12, wobei die kombinierte pharmazeutische Zusammensetzung zur Verwendung bei der Behandlung des Ewing-Sarkoms eine Zubereitung ist, die für beliebige von oralen, parenteralen, intraperitonealen, intravenösen, intraarteriellen, transdermalen, sublingualen, intramuskulären, rektalen, transbukkalen, intranasalen, inhalativen, vaginalen, intraokularen, topischen, subkutanen, intraadipösen, intraartikulären und intrathekalen Verabreichungen geeignet ist.

**14.** Pharmazeutische Zusammensetzung zur Verwendung bei der Behandlung des Ewing-Sarkoms, umfassend eine Verbindung der Formel I oder ein pharmazeutisch annehmbares Salz davon

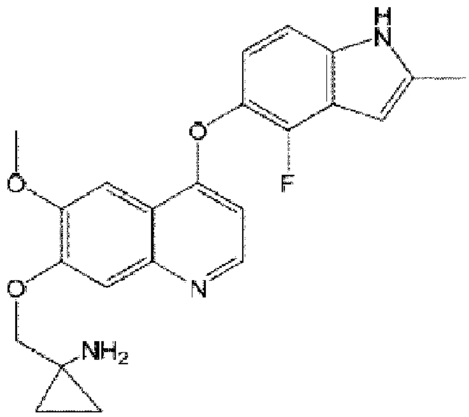

Formel I.

**15.** Pharmazeutische Zusammensetzung zur Verwendung nach Anspruch 14, wobei das Ewing-Sarkom endoskelettales Ewing-Sarkom, extraskelettales Ewing-Sarkom oder periostales Ewing-Sarkom ist; oder

das Ewing-Sarkom osteolytisches Ewing-Sarkom, sklerosierendes Ewing-Sarkom oder hybrides Ewing-Sarkom ist; oder
das Ewing-Sarkom eines ist, das nach Versagen einer vorherigen Behandlung vorliegt; bevorzugt, das Ewing-Sarkom eines ist, das nach Versagen einer Behandlung mit Strahlentherapie und/oder einem Chemotherapeutikum vorliegt.

## Revendications

**1.** Composé de formule I ou sel pharmaceutiquement acceptable de celui-ci destiné à être utilisé dans le traitement du sarcome d'Ewing,

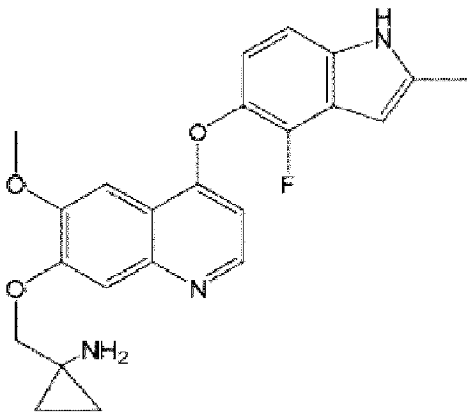

formule I.

**2.** Composé de formule I ou sel pharmaceutiquement acceptable de celui-ci destiné à être utilisé selon la revendication 1, dans lequel le sarcome d'Ewing est un sarcome d'Ewing endosquelettique, un sarcome d'Ewing extrasquelettique ou un sarcome d'Ewing périostique ; ou

le sarcome d'Ewing est un sarcome d'Ewing ostéolytique, un sarcome d'Ewing sclérosant ou un sarcome d'Ewing hybride ; ou
le sarcome d'Ewing est un sarcome qui survient après l'échec d'un traitement antérieur ; de préférence, le sarcome d'Ewing est un sarcome qui survient après l'échec d'un traitement par radiothérapie et/ou un médicament chimiothérapeutique.

**3.** Composé de formule I ou sel acceptable pharmaceutiquement de celui-ci destiné à être utilisé selon la revendication 1

ou 2, dans lequel le sel acceptable pharmaceutiquement du composé de formule I est un sel formé par le composé de formule I et l'un quelconque des acides suivants : acide chlorhydrique, acide bromhydrique, acide sulfurique, acide nitrique, acide phosphorique, acide acétique, acide trifluoroacétique, acide propionique, acide hexanoïque, acide heptanoïque, acide cyclopentane propionique, acide glycolique, acide pyruvique, acide lactique, acide malonique, acide succinique, acide malique, acide maléique, acide fumarique, acide tartrique, acide citrique, acide benzoïque, acide cinnamique, acide mandélique, acide méthanesulfonique, acide éthanesulfonique, acide 1,2-éthanedisulfonique, acide 2-hydroxyéthanesulfonique, acide benzènesulfonique, acide *p*-chlorobenzènesulfonique, acide *p*-toluènesulfonique, acide 3-phénylpropionique, acide triméthylacétique, acide *t*-butylacétique, acide dodécylsulfurique, acide gluconique, acide glutamique, acide hydroxynaphtoïque, acide salicylique et acide stéarique ; de préférence, le sel acceptable pharmaceutiquement est un hydrochlorure ou un maléate ; idéalement, le sel pharmaceutiquement acceptable est un dihydrochlorure.

**4.** Composé de formule I ou sel pharmaceutiquement acceptable de celui-ci destiné à être utilisé selon l'une quelconque des revendications 1 à 3, dans lequel le composé de formule I ou sel pharmaceutiquement acceptable de celui-ci est administré à une dose quotidienne de 3 mg à 30 mg, de préférence de 5 mg à 20 mg, mieux encore de 8 mg à 16 mg, mieux encore de 8 mg à 14 mg, et idéalement de 8 mg, 10 mg ou 12 mg ;

de préférence, le composé de formule I ou le sel pharmaceutiquement acceptable de celui-ci est administré selon un schéma intermittent de périodes de traitement et d'interruption alternées ; de préférence, le rapport entre la période de traitement et la période d'interruption en jours est de préférence de 2:0,5-2:5, mieux encore de 2:0,5-2:3, idéalement de 2:0,5-2:2, et
encore plus préférablement de 2:0,5-2:1 ; encore plus préférablement, le régime intermittent est l'un des cycles suivants : consécutivement un traitement de 2 semaines, puis une interruption de 2 semaines, consécutivement un traitement de 2 semaines, puis une interruption de 1 semaine, et consécutivement un traitement de 5 jours, puis une interruption de 2 jours, le cycle étant répété plusieurs fois.

**5.** Composé de formule I ou sel pharmaceutiquement acceptable de celui-ci destiné à être utilisé selon l'une quelconque des revendications 1 à 4, dans lequel le composé de formule I ou sel pharmaceutiquement acceptable de celui-ci destiné à être utilisé dans le traitement du sarcome d'Ewing est une préparation appropriée pour une quelconque administration orale, parentérale, intrapéritonéale, intraveineuse, intra-artérielle, transdermique, sublinguale, intramusculaire, rectale, transbuccale, intranasale, inhalée, vaginale, intraoculaire, topique, sous-cutanée, intra-adipose, intra-articulaire et intrathécale.

**6.** Composé de formule I ou sel pharmaceutiquement acceptable de celui-ci destiné à être utilisé selon l'une quelconque des revendications 1 à 5, dans lequel le composé de formule I ou sel pharmaceutiquement acceptable de celui-ci est sous forme d'une composition pharmaceutique combinée destinée à être utilisée dans le traitement du sarcome d'Ewing, comprenant un composé de formule I ou sel pharmaceutiquement acceptable de celui-ci et un second agent thérapeutique.

**7.** Composé de formule I ou sel pharmaceutiquement acceptable de celui-ci destiné à être utilisé selon la revendication 6, dans lequel le second agent thérapeutique est un médicament chimiothérapeutique et/ou un médicament antitumoral ciblé à petite molécule ;

de préférence, le médicament chimiothérapeutique est un ou plusieurs parmi un agent alkylant, un podophyllum, un analogue de camptothécine, un taxane, un antimétabolite et un antibiotique antitumoral ;
plus préférablement, le médicament chimiothérapeutique est un ou plusieurs d'un médicament à base de platine, d'un dérivé de fluoropyrimidine, d'un taxane, d'un analogue de camptothécine, d'un alcaloïde vinca, de pémétrexed, de carmustine, d'étoposide, de téniposide, de mitomycine, d'ifosfamide, de cyclophosphamide, d'azacitidine, de pirarubicine, d'amrubicine, de méthotrexate, de bendamustine, d'épirubicine, de doxorubicine, d'actinomycine D, de dactinomycine, de bléomycine, de témozolomide, de LCL-161, de KML-001, de sapacitabine, de plinabuline, de tréosulfane, de chlorhydrate de tipiracil, de $^{153}$Sm-EDTMP, de tégafur-giméracil-otéracil potassium et d'encéquidar ;
de préférence, le médicament à base de platine est un ou plusieurs parmi l'oxaliplatine, la cisplatine, la carboplatine, la nédaplatine et la dicycloplatine ; le dérivé de fluoropyrimidine est un ou plusieurs parmi la gemcitabine, la capécitabine, le fluorouracile, la difuradine, la doxifluridine, le tégafur, le carmofur et la trifluridine ; le taxane est un ou plusieurs parmi le paclitaxel, le paclitaxel lié à l'albumine et le docétaxel ; l'analogue de camptothécine est un ou plusieurs parmi la camptothécine, l'hydroxycamptothécine, l'irinotécan et le topotécan ; l'alcaloïde vinca est un ou plusieurs parmi la vinorelbine, la vinblastine, la vincristine, la vindésine et la vinflunine ;

de préférence, le médicament antitumoral ciblé à petite molécule est un inhibiteur de protéine kinase ;
plus préférablement, le médicament antitumoral ciblé à petite molécule est un inhibiteur de tyrosine kinase et un inhibiteur de sérine et/ou de thréonine kinase ;
plus préférablement, le médicament antitumoral ciblé à petite molécule est un ou plusieurs de l'erlotinib, afatinib, crizotinib, céritinib, vémurafénib, dabrafenib, cabozantinib, gefitinib, dacomitinib, osimertinib, alectinib, brigatinib, lorlatinib, trametinib, larotrectinib, icotinib, lapatinib, vandétanib, selumetinib, sorafénib, ohnutinib, savolitinib, fruquintinib, entrectinib, dasatinib, ensartinib, lenvatinib, itacitinib, pyrotinib, binimetinib, erdafitinib, axitinib, nératinib, cobimetinib, acalabrutinib, famitinib, masitinib, ibrutinib, rociletinib, nintédanib, lénalidomide, everolimus, LOXO-292, vorolanib, bémcentinib, capmatinib, entrectinib, TAK-931, ALT-803, palbociclib, L-malate de famitinib, LTT-462, BLU-667, ningetinib, tipifarnib, poziotinib, DS-1205c, capivasertib, SH-1028, diméthyldiguanide, séliciclib, OSE-2101, APL-101, berzosertib, idélalisib, lérociclib, ceralasertib, PLB-1003, tomivosertib, AST-2818, SKLB-1028, D-0316, LY-3023414, allitinib, MRTX-849, AP-32788, AZD-4205, lifirafénib, vactosertib, mivebresib, napabucasine, sitravatinib, TAS-114, molibresib, CC-223, rivocéranib, CK-101, LXH-254, simotinib, GSK-3368715, TAS-0728, masitinib, tépotinib, HS-10296, AZD-4547, mérestinib, olaptesed pégol, galunisertib, ASN-003, gédatolisib, défactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, succinate d'épitinib, tésévatinib, SPH-1188-11, BPI-15000, copanlisib, niraparib, olaparib, véliparib, tosylate de talazoparib, DV-281, sirémadlin, télaglénastat, MP-0250, GLG-801, ABTL-0812, bortézomib, panobinostat, tucidinostat, vorinostat, resminostat, épacadostat, tazémétostat, entinostat, mocétinostat, et quisinostat ;
encore plus préférablement, le médicament antitumoral ciblé à petite molécule est un ou plusieurs parmi le sorafénib, l'évérolimus, l'erlotinib, l'afatinib, le crizotinib, le céritinib, le vémurafénib, le dabrafénib, le cabozantinib, le géfitinib, le dacomitinib, l'osimertinib, l'alectinib, le brigatinib, le lorlatinib, le tramétinib, le larotrectinib, l'icotinib, le lapatinib, le vandétanib, le sélumétinib, l'ohnutinib, le savolitinib, le fruquintinib, l'entrectinib, le dasatinib, l'ensartinib, le lenvatinib, l'itacitinib, le pyrotinib, le binimetinib, l'erdafitinib, l'axitinib, le nératinib, le cobimétinib, l'acalabrutinib, le famitinib, le masitinib, l'ibrutinib et le nintedanib.

**8.** Composé de formule I ou sel pharmaceutiquement acceptable de celui-ci destiné à être utilisé selon l'une quelconque des revendications 6 et 7, dans lequel le second agent thérapeutique est une combinaison de vincristine, de cyclophosphamide et d'actinomycine D ; ou

le second agent thérapeutique est une combinaison de vincristine, de cyclophosphamide et de doxorubicine ; ou
le second agent thérapeutique est une combinaison de doxorubicine, de méthotrexate, de cyclophosphamide, d'actinomycine D, de bléomycine et de vincristine ; ou
le second agent thérapeutique est une combinaison de vincristine, de cyclophosphamide, d'actinomycine D et/ou de doxorubicine ; ou
le second agent thérapeutique est une combinaison d'ifosfamide et d'étoposide ; ou
le second agent thérapeutique est une combinaison de doxorubicine et de cyclophosphamide ; ou
le second agent thérapeutique est une combinaison de vincristine et de cyclophosphamide.

**9.** Composé de formule I ou sel pharmaceutiquement acceptable de celui-ci destiné à être utilisé selon l'une quelconque des revendications 6 à 8, dans lequel le second agent thérapeutique est l'un d'un régime VAC, d'un régime T9, d'un régime IE et d'un régime AC.

**10.** Composé de formule I ou sel pharmaceutiquement acceptable de celui-ci destiné à être utilisé selon l'une quelconque des revendications 6 à 9, dans lequel, dans la composition pharmaceutique combinée, le sel pharmaceutiquement acceptable est un chlorhydrate ou un maléate ; plus préférablement, le sel pharmaceutiquement acceptable est un dichlorhydrate.

**11.** Composé de formule I ou sel pharmaceutiquement acceptable de celui-ci destiné à être utilisé selon l'une quelconque des revendications 6 à 10, dans lequel le composé de formule I ou le sel pharmaceutiquement acceptable de celui-ci est administré à une dose quotidienne de 3 mg à 30 mg ;
de préférence, le composé de formule I ou le sel pharmaceutiquement acceptable de celui-ci est administré selon un schéma intermittent de périodes de traitement et d'interruption alternées ; de préférence, le rapport entre la période de traitement et la période d'interruption en jours est de préférence de 2:0,5-2:5, mieux encore de 2:0,5-2:3, idéalement de 2:0,5-2:2, et idéalement de 2:0,5-2:1 ; idéalement encore, le schéma intermittent est l'un des cycles suivants : consécutivement un traitement de 2 semaines, puis une interruption de 2 semaines, consécutivement un traitement de 2 semaines, puis une interruption de 1 semaine, et consécutivement un traitement de 5 jours, puis une interruption

de 2 jours, le cycle étant répété plusieurs fois.

**12.** Composé de formule I ou sel pharmaceutiquement acceptable de celui-ci destiné à être utilisé selon l'une quelconque des revendications 6 à 11, dans lequel le composé de formule I ou le sel pharmaceutiquement acceptable de celui-ci et le second agent thérapeutique sont administrés simultanément, séquentiellement ou non simultanément.

**13.** Composé de formule I ou sel pharmaceutiquement acceptable de celui-ci destiné à être utilisé selon l'une quelconque des revendications 6 à 12, dans lequel la composition pharmaceutique combinée destinée à être utilisée dans le traitement du sarcome d'Ewing est une préparation appropriée pour une quelconque administration orale, parentérale, intrapéritonéale, intraveineuse, intra-artérielle, transdermique, sublinguale, intramusculaire, rectale, transbuccale, intranasale, inhalée, vaginale, intraoculaire, topique, sous-cutanée, intra-adipose, intra-articulaire et intrathécale.

**14.** Composition pharmaceutique destinée à être utilisée dans le traitement du sarcome d'Ewing, comprenant un composé de formule I ou un sel pharmaceutiquement acceptable de celui-ci

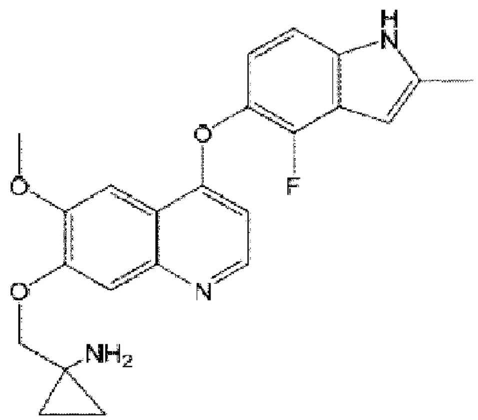

formule I.

**15.** Composition pharmaceutique destinée à être utilisée selon la revendication 14, dans laquelle le sarcome d'Ewing est un sarcome d'Ewing endosquelettique, un sarcome d'Ewing extrasquelettique ou un sarcome d'Ewing périostique ; ou

le sarcome d'Ewing est un sarcome d'Ewing ostéolytique, un sarcome d'Ewing sclérosant ou un sarcome d'Ewing hybride ; ou

le sarcome d'Ewing est un sarcome qui survient après l'échec d'un traitement antérieur ; de préférence, le sarcome d'Ewing est un sarcome qui survient après l'échec d'un traitement par radiothérapie et/ou un médicament chimiothérapeutique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008112407 A
- CN 102344438 A


### Non-patent literature cited in the description


- **DENG**. *Chinese General Practice*, 2014, vol. 17 (12)
- **PRETZ et al.** *INTERNATIONAL JOURNAL OF RADIATION: ONCOLOGY BIOLOGY PHYSICS*, 2014, vol. 90 (1)